# FASCICULE DE BREVET EUROPEEN

(11) **EP 1 618 923 B1**
(45) Date de publication et mention de la délivrance du brevet: **14.02.2007**
(21) Numéro de dépôt: 05291566.7
(22) Date de dépôt: 21.07.2005
(51) Int. Cl.: A61N 1/37

(54) **Stimulateur cardiaque implantable à détection automatique de la mise en place d'une sonde et de l'implantation du boîtier**
Implantable cardiac stimulation device with automatic detection of the installation of a probe and of the implantation of the housing
Implantierbarer Herzschrittmacher mit automatischer Detektion des Installieren einer Sonde und der Implantation des Gehäuses

(30) Priorité: 23.07.2004 FR 0408162
(43) Date de publication de la demande: 25.01.2006
(73) Titulaire: ELA MEDICAL, 92541 Montrouge (FR)
(72) Inventeur: Legay, Thierry, 91640 Fontenay les Briis (FR); Decoene, Dominique, 78760 Jouars Pontchartrain (FR)
(74) Mandataire: Dupuis-Latour, Dominique

(56) Documents cités:
- EP-A- 1 438 985
- US-A- 5 370 666
- US-A- 5 522 856
- US-A- 5 534 018
- US-A- 6 016 447

## Description

L'invention concerne les "dispositifs médicaux implantables actifs" tels que définis par la Directive 90/385/CEE du 20 juin 1990 du Conseil des communautés européennes, notamment les dispositifs stimulateurs cardiaques, défibrillateurs, cardioverteurs et/ou dispositifs "multisite" permettant de délivrer au coeur des impulsions électriques de faible énergie pour le traitement des troubles du rythme cardiaque.

Ces dispositifs comportent un générateur contenant dans un même boîtier les divers circuits électroniques et leur pile d'alimentation. Au moment de l'implantation, ce générateur est relié mécaniquement et connecté électriquement à une sonde pourvue d'électrodes de stimulation intracardiaque permettant de détecter les potentiels de dépolarisation du myocarde et de délivrer à ce dernier les impulsions de stimulation produites par le générateur.

Le branchement de la sonde doit pouvoir être détecté automatiquement par le générateur, de manière à activer diverses fonctionnalités, lancer des algorithmes, initialiser un certain nombre de paramètres, mémoriser des données de départ, etc.

Les US-A-5 522 856 et US-A-5 370 666 détectent une telle insertion de la sonde par scrutation en continu des bornes de la tête de connecteur du dispositif et mesure de l'impédance entre ces bornes : en l'absence de sonde, cette impédance est extrêmement élevée, mais dès l'insertion d'une sonde la valeur décroît au dessous d'un certain seuil dont le franchissement est détecté pour sortir le stimulateur d'un mode de veille et le placer dans un mode totalement fonctionnel.

La scrutation continue de l'impédance est cependant pénalisante en termes de consommation et donc de durée de vie de la pile, car elle nécessite à chaque mesure l'injection d'un courant entre les bornes de la sonde et l'activation de circuits de mesure de la tension correspondante recueillie.

Une autre possibilité consiste à recourir à un programmateur externe pour activer le générateur en fin d'implantation, mais cette technique nécessite l'intervention du chirurgien, donc une manipulation et un risque d'oubli.

L'un des buts de l'invention est de proposer un circuit qui puisse détecter de façon entièrement automatique le branchement d'une sonde, mais sans nécessiter de mesure directe et permanente de l'impédance entre les bornes, et sans recours à un programmateur externe.

D'autre part, il est généralement possible de relier à un même générateur deux types de sonde différents, monopolaire ou bipolaire, au choix du chirurgien selon le type de pathologie à traiter. Dans le cas d'une sonde monopolaire (ou "unipolaire"), la détection et la stimulation sont opérées entre l'électrode unique et le boîtier métallique du générateur, tandis que dans une sonde bipolaire, détection et stimulation peuvent effectuées soit en mode différentiel entre deux électrodes de la sonde, soit en mode commun entre le boîtier du générateur et l'une ou l'autre des électrodes.

Bien entendu, de nombreux paramètres internes du générateur doivent être choisis en fonction du type de sonde utilisée, monopolaire ou bipolaire : commutation des bornes devant être utilisées, recueil des signaux de dépolarisation, ajustement des paramètres de stimulation, modification des algorithmes de pilotage du microprocesseur, etc. On comprendra que toute erreur dans la sélection du type de fonctionnement (monopolaire ou bipolaire) peut entraîner des conséquences extrêmement graves : par exemple, si le dispositif est programmé pour une stimulation bipolaire mais qu'il est équipé d'une sonde monopolaire, cette erreur va provoquer une perte de capture et l'application d'une stimulation inappropriée, avec un risque majeur pour le patient.

Un autre but de l'invention est de proposer un dispositif qui puisse, de manière entièrement autonome, non seulement détecter le branchement d'une sonde, mais accessoirement déterminer aussi le type de sonde utilisée - monopolaire ou bipolaire - et commuter et paramétrer en conséquence les divers circuits et algorithmes du dispositif. Cette fonction, gérée de façon entièrement automatique par le dispositif, permet d'éviter tout risque d'erreur résultant d'un défaut de conformité entre le type de sonde utilisée et le mode de fonctionnement du dispositif (on évite ainsi, par exemple, tout risque de stimulation bipolaire appliquée par erreur à une sonde monopolaire).

La détermination automatique du type de polarité permet aussi de tenir compte du fait que dans la plupart des pays le stimulateur est livré par défaut en configuration unipolaire, pour être sûr de provoquer en tout état de cause une stimulation. Mais aujourd'hui dans la majeure partie des cas c'est une sonde bipolaire qui est implantée, ce qui nécessite de paramétrer le générateur en conséquence après y avoir raccordé la sonde. Le système selon l'invention, par détermination automatique de la polarité à utiliser à l'implantation du dispositif, autorise un paramétrage automatique du générateur sans intervention du chirurgien.

Un autre but encore de l'invention est de proposer un dispositif permettant de prendre en compte la configuration particulière où la sonde a été implantée puis connectée au boîtier du stimulateur, mais où ce dernier n'a pas encore été introduit dans le site d'implantation (l'incision ou "poche" dans laquelle le chirurgien prévoit de placer le générateur d'impulsions). Le boîtier n'est donc pas encore en contact avec les tissus du patient et ne peut pas servir de référence de potentiel : dans ce cas, il est préférable d'éviter une stimulation monopolaire sur la sonde, car les impulsions délivrées seraient inefficaces du fait de l'absence de retour de masse. Toutefois, durant cette phase intermédiaire, il est souhaitable de délivrer une stimulation bipolaire, si une sonde bipolaire ventriculaire est implantée et déjà connectée.

On verra que le dispositif de l'invention permet de prendre en compte cette configuration intermédiaire, jusqu'à implantation effective du dispositif. Ce n'est qu'après détection de l'implantation effective que seront activées les fonctions correspondantes du stimulateur, et éventuellement configuré la polarité, monopolaire ou bipolaire. Grâce à l'activation automatique des fonctionnalités du stimulateur, le chirurgien n'aura jamais besoin de forcer un mode de stimulation particulier préalablement à l'implantation du générateur dans la poche, ou après achèvement de l'implantation ; il lui suffira de connecter la sonde, quel que soit son type, au générateur, et la sécurité sera automatiquement assurée pendant et après l'intervention.

Pour atteindre les divers buts précités, l'invention propose un dispositif du type général connu par exemple d'après les US-A 5 522 856 et US-A-5 370 666 précités, c'est-à-dire comprenant un boîtier et, dans ce boîtier, un générateur et une pile d'alimentation du générateur, le générateur : étant apte à produire des impulsions de stimulation en mode monopolaire ou en mode bipolaire ; étant relié à une tête de connecteur pourvue d'au moins deux bornes aptes à être reliées à des électrodes d'une sonde de détection et de stimulation reliée au connecteur, cette sonde pouvant être une sonde monopolaire ou une sonde bipolaire ; et incluant des moyens de détection de la présence d'une sonde reliée au connecteur.

De façon caractéristique de l'invention, lesdits moyens de détection comprennent comprennent, en combinaison : des moyens de scrutation de la consommation de la pile par le générateur, aptes à mesurer le courant débité par la pile et délivrer une valeur mesurée du courant ; des moyens comparateurs, aptes à comparer la valeur mesurée à une valeur préprogrammée de seuil de courant et à indiquer que la sonde est reliée au connecteur lorsque la valeur mesurée dépasse ladite valeur préprogrammée de seuil de courant ; et des moyens aptes, lorsque la valeur mesurée ne dépasse pas ladite valeur préprogrammée de seuil de courant, à (i) détecter et compter les dépolarisations spontanées recueillies entre lesdites bornes de la tête de connecteur, (ii) comparer le nombre de dépolarisations ainsi comptées à un seuil de comptage préprogrammé, et (iii) délivrer une signal de suspicion d'implantation en cas de franchissement de ce seuil de comptage.

Les sous-revendications visent diverses formes de réalisation subsidiaires avantageuses.

On va maintenant décrire un exemple de mise en oeuvre du dispositif de l'invention, en référence aux dessins annexés.
La figure 1 est un synoptique de la cellule de mesure de la consommation incorporée au dispositif selon l'invention.
La figure 2 est un exemple de consommation relevée, variant en fonction de diverses valeurs d'impédance des sondes de stimulation.
La figure 3 est un exemple de consommation relevée, variant en fonction de la fréquence des ondes de dépolarisation détectées.
La figure 4 est un organigramme des différentes étapes permettant la détection de l'implantation du dispositif.
La figure 5 est un organigramme des différentes étapes permettant d'assurer la configuration de polarité des circuits de stimulation et de détection.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple les dispositifs implantables commercialisés par ELA Médical, Montrouge, France tels que les appareils *Symphony* et *ELA Rhapsody.*

Il s'agit de dispositifs à microprocesseur programmables comportant des circuits pour recevoir, mettre en forme et traiter des signaux électriques recueillis par des électrodes cardiaques, et délivrer des impulsions de stimulation à ces électrodes. Il est possible d'y implanter en mémoire des logiciels qui seront exécutés pour mettre en oeuvre les fonctions de l'invention décrites ci-dessous. L'adaptation de ces appareils est à la portée de l'homme du métier, et elle ne sera pas décrite en détail.

La description qui va suivre concemera essentiellement la mise en oeuvre de la fonction de stimulation ventriculaire. En effet, même dans le cas d'un appareil double chambre, la détection de l'implantation a vocation à s'assurer prioritairement que la stimulation ventriculaire est possible et effective. Si seule la stimulation auriculaire était possible et effective, l'implantation du dispositif ne serait pas automatiquement considérée comme établie, car la contribution de l'oreillette dans un dispositif double chambre est moindre. Toutefois, tout ce qui va être décrit dans le cadre de la stimulation ventriculaire est directement transposable à la stimulation auriculaire. De même, dans le cas d'un dispositif multisite, la même séquence d'étapes est semblablement applicable à chacun des étages.

Le principe de l'invention repose sur l'analyse permanente ou "scrutation" de la consommation du dispositif. Cette scrutation est permanente, y compris dans l'emballage commercial de livraison. Il est donc possible à tout instant de signaler un changement de comportement de la prothèse, révélé par un accroissement de la consommation. En particulier, l'implantation de la prothèse va faire augmenter la consommation du fait de la stimulation sur une charge qui n'est plus infinie (comme c'était le cas dans l'emballage d'expédition), de la détection de signaux cardiaques activant les filtres numériques, dont la consommation dépend du signal en entrée, ainsi que des réveils du microcontrôleur exécutant des instructions logicielles spécifiques sur chaque nouvelle détection endocavitaire.

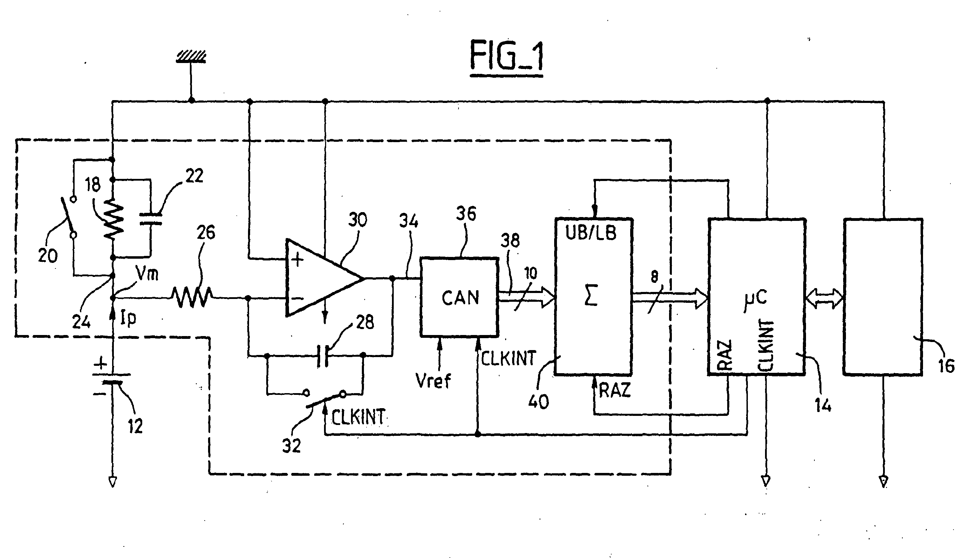

La scrutation de cette consommation est opérée par un circuit matériel dont un schéma synoptique est donné sur la figure 1. En pratique, la consommation de ce circuit est très faible, de l'ordre de 0,2 µA, valeur tout à fait acceptable même pour un fonctionnement permanent.

Ce circuit 10 est reliée d'une part à la pile 12 dont il va mesurer le courant Iₚ et, d'autre part, au microcontrôleur 14, qui est d'un type conventionnel, permettant d'exécuter le logiciel de mise en oeuvre du dispositif et comportant des mémoires morte et vive, des temporisateurs, des contrôleurs DMA, un système d'interruption, une horloge, etc. Ce microcontrôleur 14 est lui-même interfacé à un circuit 16 contenant l'électronique dédiée du dispositif avec ses étages de stimulation, amplificateurs de détection, chaînes de traitement des capteurs, système de télémétrie, références de tension, de courant et de temps, etc.

La pile 12 alimente l'intégralité des circuits électroniques du dispositif et fournit également l'énergie des impulsions de stimulation. Le courant Iₚ fourni par cette pile 12 traverse la résistance de mesure 18 lorsque la scrutation du courant est activée, c'est-à-dire lorsqu'un interrupteur 20 est ouvert (s'il n'y a plus lieu d'effectuer cette scrutation, par exemple après implantation du dispositif, le commutateur 20 sera fermé et la circuiterie de la cellule 10 désalimentée). Le condensateur 22, en parallèle sur la résistance 18, sert à filtrer les transitoires de consommation rapide traversant cette résistance.

Le courant Ip passant dans la résistance 18 produit une tension Vₘ proportionnelle au courant en 24. Cette tension est intégrée par le circuit constitué de la résistance 26, du condensateur 28 et de l'amplificateur opérationnel 30. La constante de temps d'intégration est choisie relativement courte, typiquement de 50 ms afin d'éviter une saturation de l'amplificateur 30. Le cycle d'intégration est défini par le signal CLKINT produit par le microcontrôleur 14 qui commande un interrupteur 32. Pendant la phase d'intégration, l'interrupteur 32 est ouvert et, au terme de l'intégration, la tension intégrée, présente en 34 en sortie de l'amplificateur 30, est convertie en une valeur numérique par le convertisseur analogique/numérique 36. Le commutateur 32 est ensuite fermé pour décharger le condensateur 28 avant la reprise d'un nouveau cycle d'intégration de 50 ms. Les valeurs élémentaires d'intégration sur 50 ms, délivrées en 38 en sortie du convertisseur 36, sont sommées par l'additionneur 40, pendant un temps fixé par le signal RAZ produit par le microcontrôleur 14. Cette durée de sommation est choisie suffisamment longue, typiquement de l'ordre de 6 s, pour pouvoir intégrer plusieurs cycles cardiaques du dispositif.

Au terme du temps d'intégration numérique de 6 s, le résultat de l'additionneur, sur deux octets, est lu par le microcontrôleur en deux temps (par le signal de sélection UB/LB de sélection du premier octet puis du second octet). Lorsque le microcontrôleur 14 a ainsi acquis une valeur moyenne du courant Iₚ sur la pile pendant 6 s, il réinitialise l'additionneur 40 par le signal RAZ afin de recommencer un nouveau cycle d'intégration du courant de la pile.

Une mesure de la consommation toutes les 6 s donne une représentation appropriée à la fois de la consommation globale des circuits du dispositif et de la consommation liée à la stimulation. La résolution de la mesure est choisie suffisamment faible pour permettre de discriminer la mise en marche ou l'arrêt des principaux blocs fonctionnels de la prothèse, ainsi que pour discriminer des changements dans les paramètres de stimulation. Cette résolution doit être inférieure à 1 µA, avantageusement choisie de l'ordre de 0,16 µA.

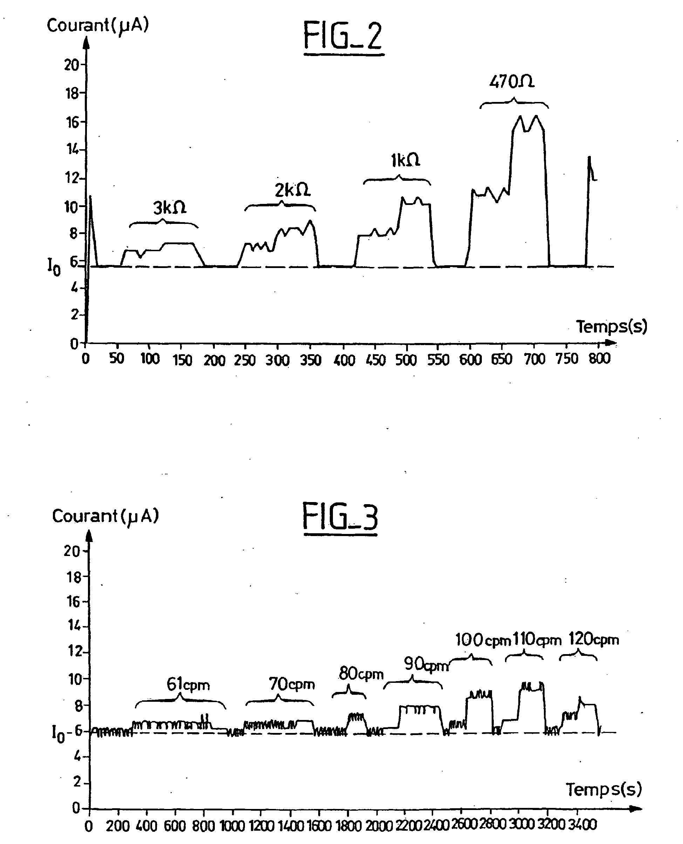

Cette résolution permet notamment de détecter la connexion d'une sonde sur le boîtier du générateur. La figure 2 montre la variation du courant pour diverses valeurs d'impédance de sonde en l'absence de signaux cardiaques détectés : quand aucune sonde n'est connectée, le courant est à une valeur de référence I₀ ; lorsqu'une sonde est connectée on peut observer deux paliers de consommation, correspondant respectivement à la connexion de la sonde ventriculaire, suivie de la connexion de la sonde auriculaire. Sur la figure 2 on a représenté les variations de courant dans le cas d'une connexion de forte impédance (3 kΩ) puis déconnexion de cette sonde et connexion d'une sonde d'impédance un peu plus faible (2 kΩ), etc. Le courant appelé est bien entendu d'autant plus élevé que l'impédance de la sonde est faible et l'on voit que la variation du courant appelé est détectable même pour des sondes de forte impédance (3 kΩ), et même si seule la sonde ventriculaire est connectée (premier palier de la variation), ce qui est notamment dans le cas d'un modèle simple chambre.

La figure 3, quant à elle, montre les variations du courant appelé en fonction de la fréquence des signaux cardiaques détectés par le dispositif, pour diverses fréquences croissantes du rythme : 61 cpm, 70 cpm, 80 cpm, ...

Par rapport à la référence de courant I₀ en l'absence de sonde, on constate que, pour les fréquences les plus faibles, l'activité des amplificateurs et filtres numériques traitant les signaux cardiaques, ainsi que les réveils du microcontrôleur exécutant les algorithmes, induisent une consommation qui est à la limite de ce que le dispositif peut détecter. Pour pallier cet inconvénient, et compenser le fait que la consommation peut rester faible en cas de stimulation inhibée (du fait de la présence d'un rythme propre du patient), l'invention prévoit de compter les dépolarisations spontanées ventriculaires (ondes R).

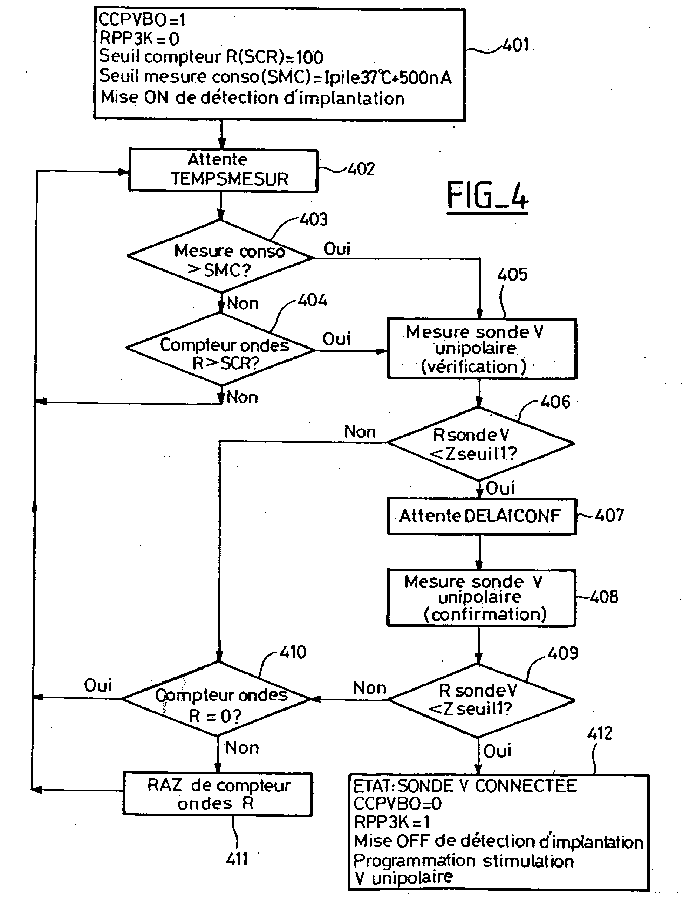

On décrira plus loin, en référence à la figure 4, ce système de comptage des ondes R, qui au surplus procure une sécurité supplémentaire. Le comptage des ondes R reste cependant optionnel, si la mesure du courant de la pile offre une résolution assez élevée pour suffire à elle seule.

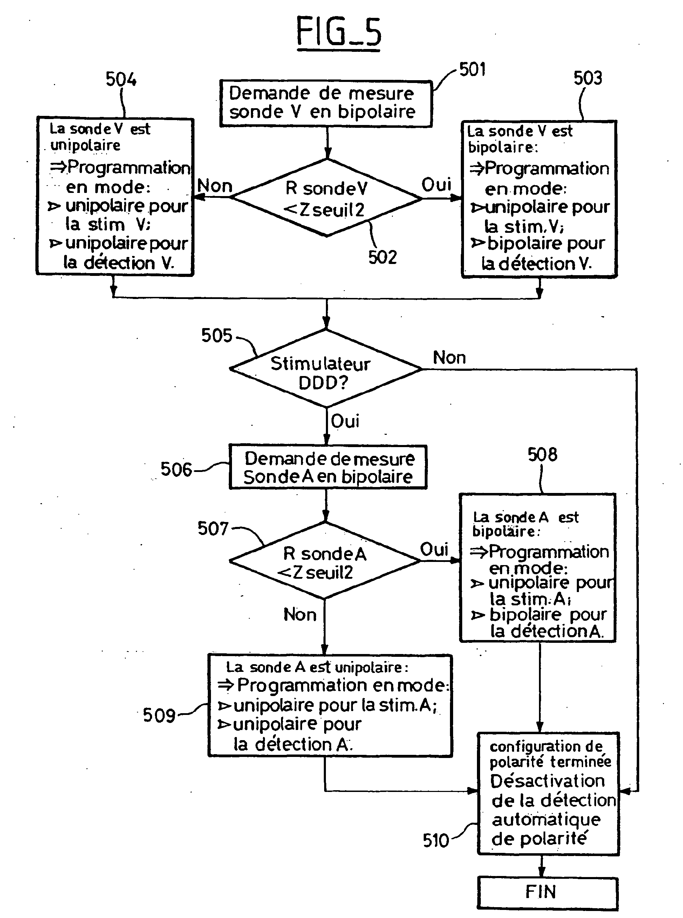

Les figures 4 et 5 sont des organigrammes détaillant les étapes successives de mise en oeuvre de l'invention, pour détecter l'implantation (figure 4) puis configurer de façon appropriée la polarité de stimulation/détection du dispositif (figure 5).

Initialement, de préférence en fin du cycle de production de l'appareil, lorsque celui-ci est dans son conditionnement stérile, un certain nombre de paramètres sont initialisés : la borne proximale ventriculaire ("RING") et la masse du boîtier ("CASE") sont court-circuités par une commande CCPVBO positionnée à 1. En court-circuitant ces électrodes, l'implant est configuré pour délivrer une stimulation simultanément en mode bipolaire et en mode monopolaire : ainsi, que le boîtier soit ou non en contact avec les tissus du patient, l'entraînement ventriculaire sera effectif dès lors qu'une sonde ventriculaire aura été implantée et raccordée à la prothèse.

Par ailleurs, un indicateur RPP3K est positionné à 0 pour indiquer que l'appareil n'est pas implanté, et des seuils programmables sont définis : comptage des ondes R (typiquement 100), et seuil de consommation SMC, correspondant à une suspicion d'implantation ; le seuil de consommation est de préférence initialisé à la valeur du courant consommé par la prothèse à 37°C sur charge infinie, augmentée d'une valeur programmable significative, révélatrice d'une implantation éventuelle (typiquement 500 nA). Le cycle de détection d'implantation est alors lancé ("ON").

Après ces étapes d'initialisation 401, le dispositif exécute une temporisation de durée supérieure ou égale à la durée d'une mesure du courant de la pile, par exemple une temporisation de 6,25 s (étape 402).

A l'étape 403, la valeur de consommation mesurée est comparée au seuil de consommation SMC :
- si le seuil est dépassé, alors une mesure d'impédance de sonde est effectuée à titre de vérification, en mode monopolaire pour le ventricule (étape 405),
- dans le cas contraire, un test est opéré à l'étape 404 pour surveiller la survenue d'ondes R ; si le nombre de détections dépasse le seuil SCR, laissant suspecter une implantation du fait du recueil de ces ondes, alors l'étape 405 de vérification est effectuée. Dans le cas contraire, une nouvelle itération est engagée (retour à la temporisation 402 dans l'attente d'une nouvelle mesure du courant consommé).

La valeur d'impédance mesurée à l'étape 405 est comparée à une valeur discriminatoire Zₛₑᵤᵢₗ₁, par exemple 2 kΩ, correspondant à la valeur d'impédance la plus élevée susceptible d'être rencontrée (étape 406) :
- si le seuil Zₛₑᵤᵢₗ₁ est franchi, une implantation est suspectée, qui devra être confirmée par les étapes 407, 408, 409 et 412,
- dans le cas contraire, le compteur d'ondes R est éventuellement remis à zéro après vérification de sa valeur (étapes 410, 411) et une nouvelle itération est engagée (retour à l'étape 402),

Si la vérification effectuée aux étapes 405 et 406 se révèle positive, il convient d'effectuer une vérification avant de décider si la prothèse est réellement connectée à ces sondes et donc implantée. Cette confirmation doit cependant être effectuée après une temporisation (étape 407) permettant au praticien qui aurait connecté la sonde et placé le boîtier dans la poche d'implantation de réintervenir rapidement pour affiner les conditions d'implantation (position des sondes, du boîtier, ligatures, etc.), par exemple pendant 5 minutes.

Après la temporisation 407, une mesure d'impédance de sonde est effectuée (étape 408) pour confirmer que le boîtier et une sonde ventriculaire monopolaires sont bien en place et connectés correctement. Le critère de discrimination (étape 409) est identique au précédent (étape 406) : si l'implantation et la connexion de la sonde sont confirmés, alors (étape 412) :
- le court-circuit entre l'électrode proximale ventriculaire et la masse du boîtier est supprimé (CCPVPO = '0') et l'indicateur d'implantation est positionné (RPP3K ='1'),
- l'algorithme de détection d'implantation est désactivé ("OFF") entraînant la fermeture de l'interrupteur 20 (figure 1), et
- la stimulation ventriculaire est programmée en mode monopolaire.

La phase suivante, illustrée figure 5, consiste à rechercher la polarité des sondes présentes, pour choisir, préférentiellement, une configuration de détection bipolaire si une sonde bipolaire est connectée, et une configuration de stimulation monopolaire par défaut.

La recherche de polarité commence par la sonde ventriculaire (étapes 501 à 504) puis, si l'appareil est un double chambre (étape 505), continue par la recherche de polarité de la sonde auriculaire (étapes 506 à 510).

L'étape 501 est une mesure d'impédance de la sonde ventriculaire en mode bipolaire, c'est-à-dire entre l'électrode proximale ("RING") et l'électrode distale ("TIP").

Le résultat est comparé (étape 502) à un seuil Zₛₑᵤᵢₗ₂, de préférence choisi inférieur ou à égal à Zₛₑᵤᵢₗ₁ (une valeur Zₛₑᵤᵢₗ₂ = 2,5 kΩ permet par exemple de prendre en compte le fait que l'impédance bipolaire peut être supérieure à l'impédance monopolaire). Si la valeur mesurée est inférieure à Zₛₑᵤᵢₗ₂, alors on considère qu'on est en présence d'une sonde bipolaire (étape 503) et la détection ventriculaire est configurée pour être en mode bipolaire. De préférence, on choisit de maintenir la stimulation ventriculaire en mode monopolaire, ce qui constitue une sécurité maximale pour le patient et le médecin (qui, ultérieurement, pourra toujours forcer la programmation en mode bipolaire).

Dans le cas contraire, la sonde est une sonde monopolaire (étape 504) et la stimulation et la détection sont programmées en mode monopolaire.

L'étape 505 teste si le modèle de l'appareil est du type double chambre, car dans ce cas, il y a également lieu de configurer l'étage auriculaire (étapes 506 à 509, homologues des étapes 501 à 504).

À l'étape 510, la configuration automatique du dispositif est achevée, et l'algorithme est désactivé.

Pour un système multisite, cette étape 510 serait remplacée par la scrutation d'un autre étage suivant le même principe, pour vérifier à quel type de sonde il est raccordé puis le configurer automatiquement.

## Revendications

1. Un dispositif médical implantable actif, notamment un stimulateur cardiaque, défibrillateur, cardioverteur et/ou dispositif multisite permettant de délivrer au coeur des impulsions électriques de faible énergie pour le traitement des troubles du rythme cardiaque,
ce dispositif comprenant un boîtier et, dans ce boîtier, un générateur (14, 16) et une pile (12) d'alimentation du générateur, le générateur :
- étant apte à produire des impulsions de stimulation en mode monopolaire ou en mode bipolaire,
- étant relié à une tête de connecteur pourvue d'au moins deux bornes aptes à être reliées à des électrodes d'une sonde de détection et de stimulation reliée au connecteur, cette sonde pouvant être une sonde monopolaire ou une sonde bipolaire, et
- incluant des moyens de détection de la présence d'une sonde reliée au connecteur,
dispositif **caractérisé en ce que** lesdits moyens de détection comprennent, en combinaison :
- des moyens de scrutation de la consommation de la pile par le générateur, aptes à mesurer le courant (Ip) débité par la pile (12) et délivrer une valeur mesurée du courant,
- des moyens comparateurs (14), aptes à comparer la valeur mesurée à une valeur préprogrammée de seuil de courant (SMC) et à indiquer que la sonde est reliée au connecteur lorsque la valeur mesurée dépasse ladite valeur préprogrammée de seuil de courant, et
- des moyens aptes, lorsque la valeur mesurée ne dépasse pas ladite valeur préprogrammée de seuil de courant, à :
**·** détecter et compter les dépolarisations spontanées recueillies entre lesdites bornes de la tête de connecteur,
· comparer le nombre (Compteur Ondes R) de dépolarisations ainsi comptées à un seuil de comptage (SCR) préprogrammé, et
· délivrer une signal de suspicion d'implantation en cas de franchissement de ce seuil de comptage.

2. Le dispositif de la revendication 1, dans lequel les moyens de scrutation de la consommation comprennent des moyens intégrateurs (26, 28, 30), aptes à délivrer à intervalles réguliers une succession de valeurs mesurées représentatives de la valeur, intégrée pendant une durée d'intégration prédéterminée, du courant débité par la pile.

3. Le dispositif de la revendication 1, dans lequel les moyens de scrutation de la consommation comprennent des moyens convertisseurs analogique/ numérique (36), aptes à délivrer ladite valeur mesurée sous forme d'un mot numérique.

4. Le dispositif de la revendication 3, dans lequel les moyens de mesure comprennent des moyens sommateurs (40), aptes à délivrer la valeur mesurée sous forme d'un cumul opéré pendant une durée de sommation prédéterminée.

5. Le dispositif de la revendication 1, comprenant en outre des moyens de test d'impédance, conditionnellement activés en cas de franchissement du seuil de courant par la valeur mesurée, aptes à mesurer l'impédance de sonde entre lesdites bornes de la tête de connecteur, à comparer la valeur (RsondeV) de l'impédance ainsi mesurée à un seuil d'impédance préprogrammé (Zseuil1), et à délivrer une signal de suspicion d'implantation en cas d'impédance mesurée inférieure à ce seuil d'impédance.

6. Le dispositif de la revendication 5, dans lequel les moyens de scrutation comportent en outre des moyens de contrôle, déclenchés en réponse à la délivrance du signal de suspicion d'implantation, et aptes à :
- initier une temporisation (DELAICONF),
- à la fin de la temporisation, réitérer l'activation des moyens de test d'impédance, et
- en cas de test délivrant à nouveau un signal de suspicion, délivrer un signal de confirmation d'implantation (RPP3K ='1').

7. Le dispositif de la revendication 6, dans lequel les moyens de scrutation comportent en outre des moyens, déclenchés en réponse à la délivrance d'un signal de confirmation d'implantation, aptes à faire passer le générateur d'un mode de stimulation à la fois monopolaire et bipolaire, en un mode de stimulation monopolaire seul.

8. Le dispositif de la revendication 6 ou 7, dans lequel les moyens de scrutation comportent en outre des moyens, déclenchés en réponse à la délivrance d'un signal de confirmation d'implantation, aptes à détecter le type de sonde, monopolaire ou bipolaire, dont les électrodes sont reliées auxdites deux bornes au moins.

9. Le dispositif de la revendication 8, dans lequel les moyens aptes à détecter le type de sonde, monopolaire ou bipolaire, comprennent des moyens de mesure d'impédance de sonde entre lesdites deux bornes au moins de la tête de connecteur, et des moyens aptes à comparer la valeur (RsondeV) de l'impédance ainsi mesurée à un seuil préprogrammé (Zseuil2) de discrimination de sonde.

10. Le dispositif de la revendication 8, comprenant en outre des moyens aptes à configurer, en réponse au type de sonde détecté, le mode de fonctionnement, monopolaire ou bipolaire, des circuits de stimulation et de détection du générateur associé à ladite sonde.

## Claims

1. An active implantable medical device, in particular a pacemaker, defibrillator, cardioverter and/or a multisite device, allowing the delivery of electrical pulses of small energy to the heart for treating cardiac arrhythmias,
this device comprising a casing, and, in this casing, a generator (14, 16) and a power supply battery (12) for the generator, the generator:
- being capable of producing stimulation pulses in a monopolar mode or a bipolar mode,
- being connected to a connector head equipped with at least two terminals capable of being connected to electrodes of a detection and stimulation probe connected to the connector, wherein said probe may be a monopolar probe or a bipolar probe, and
- including means for detecting the presence of a probe connected to the connector,
a device **characterised in that** said detection means comprises in combination:
- means for scanning the battery consumption by the generator, capable of measuring the current (Ip) delivered by the battery (12) and delivering a measured value of the current,
- comparator means (14) capable of comparing the measured value with a pre-programmed threshold current value (SMC) and indicating that the probe is connected to the connector when the measured value exceeds said pre-programmed threshold current value, and
- means capable of, when the measured value does not exceed said pre-programmed threshold current value:
■ detecting and counting spontaneous depolarisations collected between said terminals of the connector head,
■ comparing the number (wave counter R) of depolarisations thus counted with a pre-programmed counting threshold (SCR), and
■ delivering a signal of suspicion of implantation in the event of crossing of this counting threshold.

2. The device of claim 1, wherein the consumption scanning means comprises integrator means (26, 28, 30) capable of delivering with regular intervals a succession of measured values representative of the value of the battery output current, integrated throughout a predetermined integration period.

3. The device of claim 1, wherein the consumption scanning means comprises analog/digital converter means (36) capable of delivering said measured value in the form of a digital word.

4. The device of claim 3, wherein the measuring means comprises summing means (40) capable of delivering the measured value in the form of a cumulative sum operated throughout a predetermined summation period.

5. The device of claim 1, further comprising impedance test means, conditionally activated in the event that the measured value crosses the current threshold, capable of measuring probe impedance between said connector head terminals, comparing the impedance value (RsondeV) thus measured with a pre-programmed impedance threshold (Zseuil1), and delivering a signal of suspicion of implantation in the event that the measured impedance is lower than this impedance threshold.

6. The device of claim 5, wherein the scanning means further comprises controlling means triggered in response to the delivery of the signal of suspicion of implantation, and capable of:
- initiating a temporisation (DELAICONF),
- at the end of the temporisation, reiterating activation of the impedance test means, and
- in the event of a test again delivering a signal of suspicion, delivering a signal of confirmation of implantation (RPP3K = '1').

7. The device of claim 6, wherein the scanning means further comprises means, triggered in response to the delivery of a signal confirming implantation, capable of operating the generator from a mode of simultaneous monopolar and bipolar stimulation, to a mode of monopolar stimulation only.

8. The device of claim 6 or 7, wherein the scanning means further comprises means, triggered in response to the delivery of a signal confirming implantation, capable of detecting the type of probe, monopolar or bipolar, the electrodes of which are connected at least to said two terminals.

9. The device of claim 8, wherein the means capable of detecting the type of probe, monopolar or bipolar, comprises means for measuring probe impedance between at least said two terminals of the connector head, and means capable of comparing the value (RsondeV) of the thus measured impedance with a pre-programmed threshold (Zseuil2) of probe discrimination.

10. The device of claim 8, further comprising means capable of configuring, in response to the type of probe detected, the operating mode, monopolar or bipolar, of the circuits of stimulation and detection of the generator associated with said probe.

## Patentansprüche

1. Aktive implantierbare medizinische Vorrichtung, insbesondere Herzschrittmacher, Defibrillator, Kardioverter und/oder Multissite-Vorrichtung, die es erlaubt, an das Herz elektrische Impulse geringer Energie zur Behandlung von Herzrhythmusstörungen abzugeben,
wobei diese Vorrichtung ein Gehäuse umfasst, und, in diesem Gehäuse, einen Generator (14, 16) und eine Batterie (12) zur Versorgung des Generators, wobei der Generator:
- geeignet ist, Stimulationsimpulse im monopolaren Modus oder im bipolaren Modus zu erzeugen,
- mit dem Kopf eines Verbinders verbunden ist, der mit wenigstens zwei Anschlüssen versehen ist, die geeignet sind, mit Elektroden einer mit dem Verbinder verbundenen Erfassungs- und Stimulationssonde verbunden zu werden, wobei diese Sonde eine monopolare Sonde oder eine bipolare Sonde sein kann, und
- Mittel zur Erfassung der Anwesenheit einer mit dem Verbinder verbundenen Sonde umfasst,
Vorrichtung, die **dadurch gekennzeichnet ist, dass** die Erfassungsmittel kombiniert folgendes umfassen:
- Mittel zur Untersuchung des Batterieverbrauchs durch den Generator, die geeignet sind, den durch die Batterie (12) abgegebenen Stromfluss (Ip) zu messen und einen Strommesswert abzugeben,
- Vergleichmittel (14), die geeignet sind, den gemessenen Wert mit einem vorprogrammierten Stromschwellenwert (SMC) zu vergleichen und anzugeben, dass die Sonde mit dem Verbinder verbunden ist, wenn der gemessene Wert den vorprogrammierten Stromschwellenwert übersteigt, und
- Mittel, die, wenn der gemessene Wert den vorprogrammierten Stromschwellenwert nicht übersteigt, zu folgendem geeignet sind:
■ Erfassung und Zählung der spontanen Depolarisationen, die zwischen den Anschlüssen des Verbinderkopfs aufgenommen werden,
■ Vergleich der Anzahl (Wellenzähler R) von so gezählten Depolarisationen mit einer vorprogrammierten Zählschwelle (SCR), und
■ Abgabe eines Signals der Vermutung einer Implantation im Falle der Durchquerung dieser Zählschwelle.

2. Vorrichtung nach Anspruch 1, bei welcher die Mittel zur Untersuchung des Verbrauchs Integriermittel (26, 28, 30) umfassen, die geeignet sind, in regelmäßigen Intervallen eine Abfolge von gemessen Werten zu liefern, die für den während einer vorbestimmten Integrationsdauer integrierten Wert des durch die Batterie gelieferten Stroms repräsentativ sind.

3. Vorrichtung nach Anspruch 1, bei welcher die Mittel zur Untersuchung des Verbrauchs Analog/Digital-Wandlermittel (36) umfassen, die geeignet sind, den gemessenen Wert in der Form eines digitalen Worts zu liefern.

4. Vorrichtung nach Anspruch 3, bei welcher die Messmittel Summiermittel (40) umfassen, die geeignet sind, den gemessenen Wert in der Form einer Summe zu liefern, die während einer vorbestimmten Summierungsdauer durchgeführt wird.

5. Vorrichtung nach Anspruch 1, weiterhin mit Impedanztestmitteln, die unter der Bedingung aktiviert werden, dass der gemessene Wert die Stromschwelle durchquert, die geeignet sind, die Impedanz der Sonde zwischen den Anschlüssen des Verbinderkopfs zu messen, den so gemessenen Impedanzwert (RsondeV) mit einer vorprogrammierten Impedanzschwelle (Zseuil1) zu vergleichen, und im Falle einer gemessenen Impedanz, die geringer ist als diese Schwellenimpedanz, ein Signal der Vermutung einer Implantation abzugeben.

6. Vorrichtung nach Anspruch 5, bei welcher die Untersuchungsmittel außerdem Steuermittel umfassen, die in Antwort auf die Abgabe des Signals der Vermutung einer Implantation ausgelöst werden, und geeignet sind:
- eine Verweilzeit (DELAICONF) zu initiieren,
- am Ende der Verweilzeit die Aktivierung der Impedanztestmittel zu wiederholen, und
- im Falle eines Test, der erneut ein Vermutungssignal liefert, ein Signal der Bestätigung einer Implantation (RPP3K = '1') abzugeben.

7. Vorrichtung nach Anspruch 6, bei welcher die Untersuchungsmittel außerdem Mittel umfassen, die in Antwort auf die Abgabe eines Signals der Bestätigung einer Implantation ausgelöst werden, die geeignet sind, den Generator von einem sowohl monopolaren als auch bipolaren Stimulationsmodus in einen nur monopolaren Stimulationsmodus zu überführen.

8. Vorrichtung nach Anspruch 6 oder 7, bei welcher die Untersuchungsmittel außerdem Mittel umfassen, die in Antwort auf die Abgabe eines Signals der Bestätigung einer Implantation ausgelöst werden, die geeignet sind, den Sondentyp, monopolar oder bipolar, zu erfassen, dessen Elektroden mit wenigstens den zwei Anschlüssen verbunden sind.

9. Vorrichtung nach Anspruch 8, bei welcher die Mittel, die geeignet sind, den Sondentyp, monopolar oder bipolar, zu erfassen, Mittel zur Messung der Sondenimpedanz zwischen den zwei Anschlüssen wenigstens des Verbinderkopfs umfassen, und Mittel, die geeignet sind, den so gemessenen Impedanzwert (RsondeV) mit einer vorprogrammierten Schwelle (Zseuil2) zur Sondenunterscheidung zu vergleichen.

10. Vorrichtung nach Anspruch 8, weiterhin mit Mitteln, die geeignet sind, in Antwort auf den erfassten Sondentyp den Betriebsmodus, monopolar oder bipolar, der Stimulations- und Erfassungsschaltungen des der Sonde zugeordneten Generators zu konfigurieren.
